# EUROPEAN PATENT APPLICATION

(11) **EP 3 795 059 A1**
(43) Date of publication of application: **24.03.2021**
(21) Application number: 20197600.8
(22) Date of filing: 22.09.2020
(51) Int. Cl.: A61B 1/00, A61B 17/221

(54) **GUIDE DEVICE AND METHOD OF USING SAME**

(30) Priority: 23.09.2019 US 201962904083 P
(71) Applicant: Cook Medical Technologies LLC, Bloomington, IN 47404 (US)
(72) Inventor: ROBERTS, Erin, Bloomington, Indiana 47403 (US); JIMENEZ-RIOS, Jorge L., Bloomington, Indiana 47401 (US); HUNDLEY, Lyle, Bloomington, Indiana 47403 (US); SMITH, Johnny P., Worthington, Indiana 47471 (US); SHEETS, Jonathan, Bloomington, Indiana 47401 (US); DOW, Tyler, Martinsville, Indiana 46151 (US); AMAN, Mitchell T., Canton, Ohio 44708 (US); BUNCH, Kristen M., Bloomington, Indiana 47408 (US)
(74) Representative: Leach, Sean Adam

(57) **Abstract**

A device for guiding an elongate body into a channel of a scope may include a main body extending between a proximal end portion and a distal end portion and a lumen extending through the main body. The device may be slidably attached onto the elongate body such that the elongate body may be threaded through the lumen of the device, out of the distal end portion of the main body of the device, and into the channel of the scope. When a distal end of the elongate body reaches a desired location inside the channel, the device may be moved along the elongate body from a first position to a second position, where the device may be releasably coupled with respect to the elongate body.

## Description

### CROSS-REFERENCE TO RELATED APPLICATIONS

This application claims priority U.S. Provisional Application Ser. No. 62/904,083, filed September 23, 2019.

### TECHNICAL FIELD

The present disclosure relates to a strain relief device and method for medical catheter and/or stone retrieval assemblies.

### BACKGROUND

Various organs and passages in the body are subject to the development of stones, calculi and the like. Elongate devices are in common use today for removal of objects such as stones, calculi, concretions, foreign bodies and the like from the urinary, biliary, vascular or other systems. An elongate device generally includes a hollow, flexible sheath and a plurality of wires positioned in but extendable from one end of the sheath. The wires are joined or arranged so as to form a means, such as a basket or forceps for engaging the object. A handle may be connected to the other end of the sheath and a strain relief may be attached to the proximal portion of the sheath at the junction of the handle and the sheath to prevent the sheath from bending, kinking, or separation. In use, the basket is operable between a collapsed configuration for introduction into a patient and an expanded configuration for capturing the object to be retrieved when the wires are extended from the sheath. A scope is inserted into a corresponding system inside the patient such that a channel is provided for insertion of the elongate device into the system.

### BRIEF SUMMARY OF THE INVENTION

One general aspect of the present disclosure includes a device for guiding an elongate body into a channel of a scope, including a main body extending between a proximal end portion and a distal end portion; and a lumen extending through the main body between the proximal end portion and the distal end portion, where the device is configured to be slidably attached onto an elongate body such that the elongate body extends through the lumen and the device is movable between a first position and a second position with respect to the elongate body, where when the device is disposed in the first position, a distal end of the elongate body is configured to be capable of being threaded into the lumen, out of the distal end portion of the main body, and into a channel of a scope, so that the distal end of the elongate body reaches a desired location inside the channel, and where when the distal end of the elongate body reaches the desired location inside the channel, the device can be moved along the elongate body to the second position, where the device is releasably coupled with respect to the elongate body.

Another general aspect of the present disclosure includes a method of guiding an elongate body into a channel of a scope using a device, including placing the device in a first position with a distal end portion of the device inserted into a proximal end of a scope and into a portion of a channel of the scope; extending an elongate body through a lumen of the device by directing a distal end of the elongate body into the lumen from a proximal end portion of the device, out of the distal end portion of the device, and further extending the elongate body into the channel of the scope; holding the device in the first position as the elongate body continues to extend along the channel; moving the device with respect to the elongate body to a second position when the distal end of the elongate body reaches a desired location inside the channel; and coupling the device with respect to the elongate body.

Another general aspect of the present disclosure includes a device for guiding an elongate body into a channel of a scope, including a main body extending between a proximal end portion and a distal end portion; and a lumen extending through the main body between the proximal end portion and the distal end portion, where the device is configured to be slidably attached onto an elongate body such that the elongate body extends through the lumen and the device is movable between a first position and a second position with respect to the elongate body, where when the device is disposed in the first position, a distal end of the elongate body is configured to be capable of being threaded into the lumen, out of the distal end portion of the main body, and into a channel of a scope, so that the distal end of the elongate body reaches a desired location inside the channel, where when the distal end of the elongate body reaches the desired location inside the channel, the device can be moved along the elongate body to the second position where the device is releasably coupled with respect to the elongate body, and where when the device is in the second position, the device is releasably coupled to a component attached to a proximal portion of the elongate body.

Other systems, methods, features and advantages of the invention will be, or will become, apparent to one with skill in the art upon examination of the following figures and detailed description. It is intended that all such additional systems, methods, features, and advantages be within the scope of the invention, and be encompassed by the following claims.

### BRIEF DESCRIPTION OF THE DRAWINGS

The present disclosure can be better understood with reference to the following drawings and description. The components in the figures are not necessarily to scale, emphasis instead being placed upon illustrating the principles of the present disclosure. Moreover, in the figures, like-referenced numerals designate corresponding parts throughout the different views.
FIG. 1 is an illustration showing a perspective view of a device in accordance with certain aspects of the present disclosure.
FIG. 2 is an illustration showing a perspective view of a scope in accordance with certain aspects of the present disclosure.
FIG. 3 is an illustration showing a side view of an elongate device in accordance with certain aspects of the present disclosure.
FIG. 4 is an illustration showing a perspective view of the device of FIG. 1 attached onto the elongate device of FIG. 3 in a first position with a first portion of the device disposed inside a channel of the scope of FIG. 2 in accordance with certain aspects of the present disclosure.
FIG. 5 is an illustration showing a perspective internal view of the device of FIG. 1 disposed in a second position with respect to the elongate device of FIG. 3 and coupled to a component attached onto the elongate device in accordance with certain aspects of the present disclosure.
FIG. 6 is an illustration showing a perspective external view of the device of FIG. 1 disposed in a second position with respect to the elongate device of FIG. 3 and coupled to a component attached onto the elongate device in accordance with certain aspects of the present disclosure.

### DETAILED DESCRIPTION

Various aspects are described below with reference to the drawings in which like elements generally are identified by like numerals. The relationship and functioning of the various elements of the aspects may better be understood by reference to the following detailed description. However, aspects are not limited to those illustrated in the drawings or explicitly described below. It also should be understood that the drawings are not necessarily to scale, and in certain instances details may have been omitted that are not necessary for an understanding of aspects disclosed herein, such as conventional material, construction, and assembly.

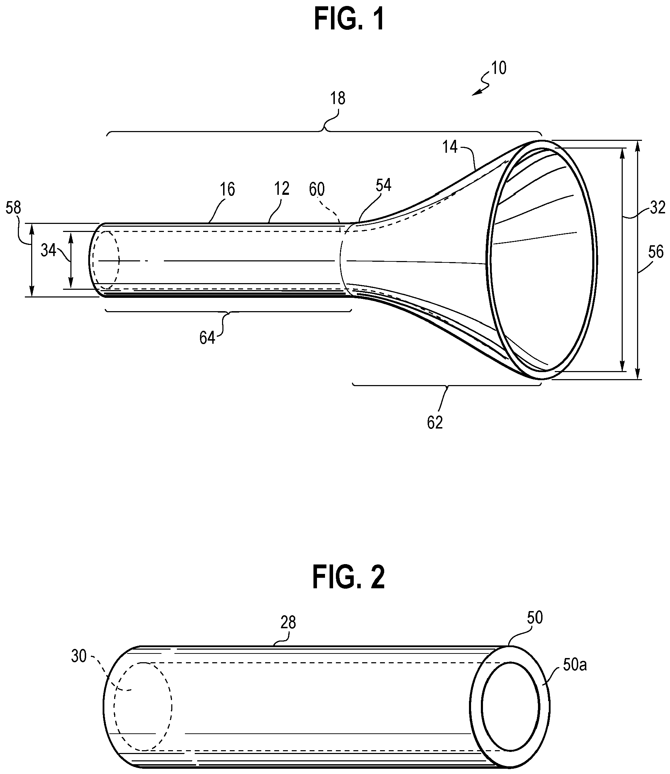

Referring to FIG. 1, a device 10 for introducing an elongate device 44, such as a retrieval device, into a channel 30 of a scope 28 (e.g., as shown in FIG. 2) is shown. While a retrieval device 44 is specifically described herein, the device 10 may be successfully implemented for use with elongate devices that are configured to extend to a remote location with a patient but may be implemented for other clinical, diagnostic, observation or other medical uses such as observation, deployment of structure, interacting with tissue in the remote location, and the like. For the sake of brevity, a device disclosed herein is described with depicted as a retrieval device, one of ordinary skill in the art, with a thorough review of the subject specification and figures, would readily comprehend how the device may be implemented for convenient passing of other types of elongate devices therethrough or for other medical uses and would comprehend which other devices might be suitable without undue experimentation.

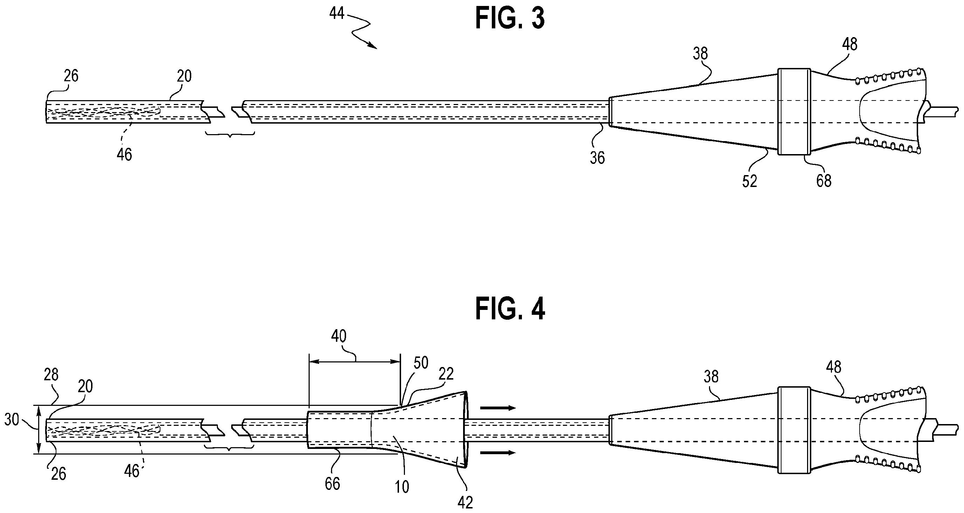

In some embodiments, as shown in FIG. 3, the elongate device 44 may include an elongate body 20 with a working component 46 disposed in a distal end 26 of the elongate body 20 and a handle 48 connected to a proximal portion 36 of the elongate body 20. In the description and drawings, the working component is a basket 46, while in other embodiments the working component 46 may be forceps, a snare, a loop, a laser fiber, an irrigation tube, or the like. In embodiments where the elongate device is a laser fiber, for example, the working component is the distal tip from which the laser is directed. A component 38 may be attached to the proximal portion 36 of the elongate body 20 with a proximal portion 52 of the component 38 coupled with the distal end 68 of the handle 48. In some embodiments, the component 38 may be a strain relief that provides support to the proximal portion 36 of the elongate body 20 and prevents the elongate body 20 from bending, kinking or separation at the junction of the elongate body 20 and the handle 48.

The device 10 may include a main body 12 extending between a proximal end portion 14 and a distal end portion 16. The device 10 may include a lumen 18 extending through the main body 12 between the proximal end portion 14 and the distal end portion 16. In some embodiments, as shown in FIG. 1, the proximal end portion 14 and the distal end portion 16 may both have a circular cross section to accommodate the configuration of the elongate body 20 that is to be slidingly received within the device 10. Additional configurations for the cross-sectional shape of the proximal end and distal end portions 14 and 16 are possible including, but not limited to, oval, square, rectangular, triangular and combinations thereof. In some embodiments, the proximal end portion 14 may be conical, and may be a geometric conical profile with a constantly changing diameter along the length of the proximal end portion 14. In other embodiments, the proximal end portion 14 may be similar to or configured as a geometric cone (i.e., with a decreasing diameter along its length) but the rate of change of diameter may not be constant (i.e., non-linear), such that a cross-section of the proximal end portion 14 forms a curve. It will be appreciated that the configuration (e.g., shape and dimension) of the cross sections of the proximal end and distal end portions 14 and 16 may be varied as needed and/or desired to accommodate the configuration of various elongate devices and/or other medical devices.

In some embodiments, the proximal end portion 14 may taper in a distal direction towards a first connection point 54 between the proximal end portion 14 and the distal end portion 16 from a first, relatively larger, outer diameter 56 to a second, relatively smaller, outer diameter 58. For example, as shown in FIG. 1, the proximal end portion 14 may have a funnel-shaped configuration. The distal end portion 16 may have a generally cylindrical shape with the substantially constant second outer diameter 58. The second outer diameter 58 may be slightly smaller than an inner diameter of the channel 30 of the scope 28 such that when the distal end portion 16 is inserted into the channel 30 of the scope 28, a relatively tight fit may be formed between the inner surface of the scope 28 and the device 10, which may minimize radial and/or axial twisting movement of the device 10 within the scope 28, thereby stabilizing the device 10 during the insertion of the elongate device 44 and providing a relatively fixed passageway for the insertion, and/or may reduce the friction or resistance to threading the elongate member 44 through the distal end portion 16 to minimize the chance of buckling or other difficulties encountered with threading the elongate member 44 through the device and into the channel 30 of the scope 28. For example, in some embodiments, the inner diameter of the channel 30 of the scope 28 may range from 1mm to 8mm, and thus the second outer diameter 58 of the device 10 may range from 0.5mm to 7.5mm.

In some embodiments, the first outer diameter 56 may be greater than an inner diameter of the channel 30 at a proximal end 50 of the scope 28 such that when the distal end portion 16 of the device 10 is inserted into the channel 30 of the scope 28, at least a portion of the proximal end portion 14 of the device 10 is disposed outside of the channel 30. The first outer diameter 56 of the device 10 may vary depending on the inner diameter of the channel 30 at the proximal end 50 of the scope 28. For example, the inner diameter of the channel 30 at the proximal end face 50a (proximal end face 50a is on the proximal end 50) of the scope 28 may be 3 FR, and thus the first outer diameter 56 may range from 6mm to 24mm. The term "about" is specifically defined herein to include the specific value referenced as well as a dimension that is within 5% of the dimension both above and below the dimension. This configuration provides a larger opening within the proximal end portion 14 of the device 10 than normally available into the channel 30 of the scope 28 for easily threading the distal end 26 of the elongate body 20 into the lumen 18 and also allows the user to easily secure the device 10 with respect to the proximal end face 50a of the scope 28.

For example, as shown in FIG. 4, when the distal end portion 16 of the device 10 is inserted into the channel 30 of the scope 28, the proximal end 50 of the scope 28 may contact the device 10 in a first position 22 along an outer surface X of the proximal end portion 14 of the device 10 where the inner diameter of the channel 30 at the proximal end 50 of the scope 28 is substantially equal to the outer diameter of the device 10. As such, a first portion 66 of the device 10 may be disposed within the channel 30 of the scope 28, and a second portion 42 of the device 10 may be disposed outside of the channel 30 of the scope 28, thereby allowing the user to easily hold the device 10 in place, against the proximal end 50 of the scope 28, in the first position 22. In some embodiments, as shown in FIG. 4, the first portion 66 may include the distal end portion 16 and at least a portion of the proximal end portion 14. It will be appreciated that the configuration (e.g., overall shape, cross-sectional shape, overall dimension, first and second outer diameters, and the way the proximal end portion tapers from the first outer diameter to the second outer diameter) of the device 10 may be varied as desired and/or necessary so as to accommodate varying configurations (e.g., shape, dimension) of the channel 30 of the scope 28.

In some embodiments, the first portion 66 of the device 10, disposed within the channel 30 of the scope 28, may have a first length 40 such that, the elongate body 20 that is to be slidingly inserted into the channel, can be guided, inside the lumen 18 of the device 10, a sufficient distance into the channel 30. For example, the first length 40 may be at least 5 cm. In some embodiments, the scope 28 may have at least one angled portion (e.g., a 45-degree angle) that the elongate body 20 needs to pass through. As such, the first length 40 may be configured such that the elongate body 20 may be guided, inside the lumen 18, through the at least one angled portion of the channel 30. Configuring a greater first length 40 will provide additional support to a greater length of the elongate body 20 as it is extended through the lumen 18. Comparing to a smaller first length 40, having a greater first length 40 will minimize the radial/axial twisting movement of the elongate body 20 within the channel 30 after the distal end 26 of the elongate body 20 has extended outside of the distal end portion 16 of the device 10, thereby minimizing damage to the distal end 26 of the elongate body 20. It will be appreciated that the length of the device 10 and the first length 40 may be varied as desired and/or necessary without departing from the scope of the invention. For example, in some embodiments, the first length 40 may be great enough such that the elongate body 20 may be guided inside the lumen 18 to pass substantially the entire length of the channel 30. This will provide the ability to direct the distal end 26 of the elongate body 20 all the way down the channel to the desired location inside the patient's body, which will avoid almost any kind of unnecessary and/or undesired movement of the elongate body 20 within the channel 30, and thereby minimizing damage to the distal end 26 of the elongate body 20.

In some embodiments, the device 10 may be made from a semi-rigid plastic material such as ABS, CLT, Polycarbonate, polyurethane, PEBAX, polyethylene, polypropylene, fluorocarbon polymers, silicone or like biocompatible polymeric materials. It will be appreciated that any suitable material may be used to form the device 10 such that the device 10 is sufficiently flexible to maneuver around the angled portions of the scope 28 disposed inside a patient's body, but also has enough column strength to prevent kinking or separation. In some embodiments, the device 10 may be extruded or injection molded. For example, the device 10 may be formed as a one-piece, over-molded component.

In some embodiments, as shown in FIG. 1, the lumen 18 at the proximal end portion 14 of the main body 12 may have a first inner diameter 32 and the lumen 18 at the distal end portion 16 of the main body 12 may have a second inner diameter 34 smaller than the first inner diameter 32. As such, the lumen 18 extending between the proximal end portion 14 and the distal end portion 16 may taper from the proximal end portion 14, in the distal direction, towards a second connection point 60 where the inner diameter is substantially similar to the second inner diameter 34, such that a tapered first portion 62 of the lumen 18 may be formed. The lumen 18 of the main body 12 extending from the second connection point 60 to the distal end portion 16 may have substantially the same inner diameter as the second inner diameter 34, such that a generally cylindrical second portion 64 of the lumen 18 may be formed. In use, as will be described in greater detail below, the first portion 62 of the lumen 18 may be configured to be coupled to the handle 48 or to the component 38 attached to the proximal portion 36 of the elongate body 20, and the second portion 64 of the lumen 18 may be configured to direct the elongate body 20 that is slidingly received therein to a desired location inside the patient.

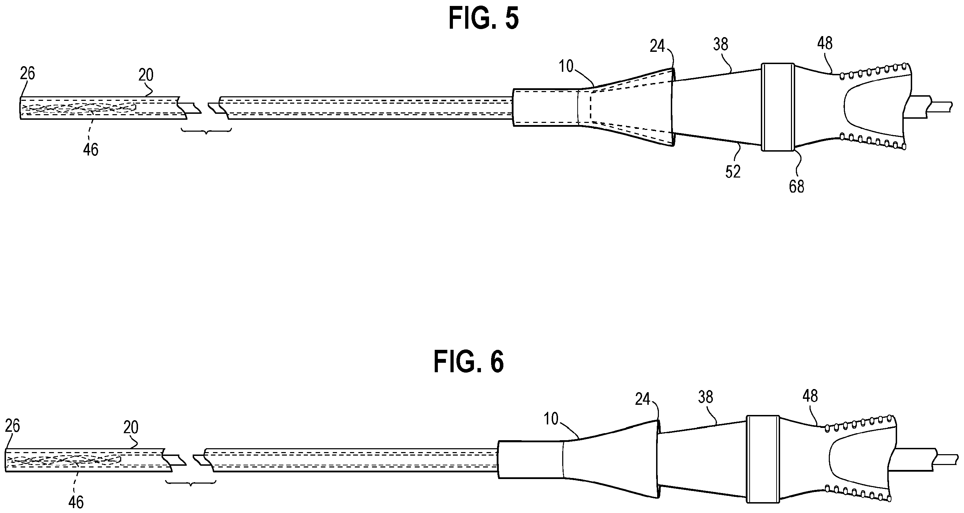

The second inner diameter 34 may be slightly greater than the outer diameter of the elongate body 20 such that the device 10 may be slidably attached onto the elongate body 20 and may be movable with respect to the elongate body 20, for example, from the first position 22 (e.g., as shown in FIG. 4) to a second position 24 (as shown in FIGs. 5 and 6). The second inner diameter 34 may vary depending on the outer diameter of the elongate body 20 that the device 10 is configured to receive. For example, the outer diameter of the elongate body 20 may range from 1.5 FR to 4.0 FR, and thus the second inner diameter 34 may range from 1.8FR to 4.3FR. When the device 10 is disposed in the first position with the distal end portion 16 of the device 10 inserted into the channel 30 of the scope 28 that has been disposed inside a patient's body, the second inner diameter 34 is configured to allow the distal end 26 of the elongate body 20 to be threaded into the lumen 18, out of the distal end portion 16 of the main body 12, and into the channel 30 of the scope 28. The difference between the second inner diameter 34 of the lumen 18 of the device 10 and the outer diameter of the elongate body 20 that the device is configured to receive may be minimized to decrease the overall outer diameter of the distal end portion 16 of the device 10, while sufficiently large to prevent binding or significant friction that might hinder sliding the elongate body 20 through the lumen 18 of the device 10. In some embodiments, a relatively tight fit between the generally cylindrical second portion 64 of the lumen 18 and the elongate body 20 may be formed, which provides support to at least a portion of the elongate body 20 so as to facilitate directing the distal end 26 of the elongate body 20 to the desired location in the patient while minimizing radial/axial twisting movement of the elongate body 20 within the lumen 18 and the channel 30. That is, the second inner diameter 34 of the generally cylindrical second portion 64 of the lumen 18 may be configured to provide the ability to straighten and stabilize the elongate body 20 prior to entry into the channel 30 of the scope 28. The first inner diameter 32 may be 300% to 600% greater than the outer diameter of the elongate body 20 such that the distal end 26 of the elongate body 20 may be easily threaded into the lumen 18. For example, in some embodiments, the first inner diameter may range from 3mm to 25mm.

In some embodiments, the device 10 may be configured such that when the device 10 is moved proximally along the elongate body 20 to the second position 24 (normally after the elongate device 44 is positioned within a patient for clinical implementation as desired), the device 10 may be releasably coupled to the component 38 attached to the proximal portion 36 of the elongate body 20. In some embodiments, the device 10 may be configured to be coupled to the handle 48. After the distal end 26 of the elongate body 20 has reached the desired location inside the patient's body, this configuration allows the user to slide the device 10 proximally and to secure the device 10 to a fixed location (e.g., coupled to the component 38 at the second position 24) such that the device 10 will not be dangling on or moving freely along the elongate body 20 when the user is trying to manipulate the elongate device 44 to extract the object at the desired location, investigate, observe, or interact with the desired location, thereby reducing the distracting effect of a dangling device 10 during use.

In the embodiments where the device 10 is configured to be coupled to the component 38, the inner configuration of the device 10 (i.e., configuration of the lumen 18), may be varied, depending on the configuration of the component 38 such that the device 10 may be releasably coupled to the component 38. In some embodiments, for example, the device 10 and the component 38 may be coupled together through a luer-lock connection or a press-fit connection. For example, when the component 38 has a generally tapered configuration (e.g., as shown in FIGs. 3 and 5), by substantially matching the inner configuration of the device 10 with the tapered configuration of the component 38, the device 10 may couple to the component 38 by inserting at least a portion of the component 38 into the lumen 18 and securely coupling them together via a luer-lock connection, tight interference, or friction fit.

It will be appreciated that any suitable connecting means may be used to couple the device 10 and the component 38 together. Although many suitable configurations and arrangements are conceivable, the configurations and arrangements must be such that the device 10 can be securely attached to the component 38 during the user's manipulation of the elongate device 44 while still remaining the ability to be separated from the component 38 for future use (e.g., insertion). In some embodiments, where the device 10 is configured to be releasably coupled to the handle 48 or the strain relief 38, the inner geometry of the proximal end portion 14 of the device 10 may include a feature or geometry that corresponds to a feature of the handle 48 or the strain relief 38 that allows for a snap fit or other releasable retaining structure (e.g., a detent/recess feature). For example, the inner surface of the proximal end portion 14 of the device 10 may have a valley that can snap over a complementary ridge on the outer surface of the handle 48.

By forming a single device 10 that can be used as both a guide device to facilitate inserting the elongate device 44 into the channel 30 of the scope 28 and a strain relief device to provide support and column strength at the junction of the elongate body 20 of the elongate device 44 and the handle 48, waste may be decreased, recyclability may be simplified, manufacturing efficiency may be increased, and manufacturing costs may be reduced.

In some embodiments, the component 38 may be a strain relief that provides support to the proximal portion 36 of the elongate body 20. As such, when the device 10 is moved back proximally and coupled to the component 38 (e.g., strain relief) attached to the proximal portion 36 of the elongate body 20, the length and flexibility of the device 10 may provide additional support and column strength to the elongate body 20 so as to prevent the elongate body 20 from kinking, twisting, or bending adjacent to the junction of the handle 48 and the proximal portion 36 of the elongate body 20. In some embodiments, the device 10 may be collapsible such that when the device 10 is coupled to the component 38, the device 10 may collapse to build additional column strength along at least a portion of the proximal portion 36 of the elongate body 20.

In some embodiments, the device 10 may be configured to be peeled away from the elongate body 20 for disposal. For example, the device 10 may include a slit or spiral cut on the main body 12 such that the device 10 may be removed from the elongate body 20 of the elongate device 44 after the device 10 has been used to direct the distal end 26 of the elongate body 20 to the desired location. The split or spiral cut is not illustrated, but will readily be understood with reference to the drawings and to the state of the art.

Before using the device 10 to direct the elongate body 20 of the elongate device 44 into the channel 30 of the scope 28, in some embodiments, the device 10 may be already provided on and stored with the elongate device 44, such that the elongate device 44 is in readiness for insertion and use by a physician. In some embodiments, the device 10 maybe stored separately from the elongate device 44 and thus a physician will need to place the device 10 onto the elongate body 20 of the elongate device 44 prior to use. To prep the device 10 for use, the physician may thread the distal end 26 of the elongate body 20 of the elongate device 44 into the lumen 18 from the proximal end portion 14 of the device 10 and out of the distal end portion 16 of the device 10 such that the device is slidably attached onto the elongate body 20 of the elongate device 44 and is movable with respect to the elongate body 20.

In use, the user may first place the scope 28 inside a patient's body (which may be inserted with the use of a guide wire) such that the channel 30 of the scope 28 may be used to direct the working component 46 of the elongate device 44 to the desired location where the object to be extracted, or the clinical area to be investigated, observed, or interacted with is located. In some embodiments where the device 10 may be already disposed upon the elongate body 20 of the elongate device 44, the user may move the device 10 distally along the elongate body 20 to the distal end 26 of the elongate body 20 and then insert the distal end portion 16 of the device into a proximal opening into the channel 30 of the scope 28 from the proximal end 50 of the scope 28 until the device 10 reaches the first position 22. In some embodiments where the device 10 is separate from the elongate device 44, before threading the elongate body 20 into the lumen 18 of the device 10, the user may first place the device 10 in the first position 22 such that the distal end portion 16 of the device 10 is inserted into the proximal end 50 of the scope 28 and into a portion of the channel 30 of the scope 28.

While holding the device 10 in place in the first position 22, the user may extend the elongate body 20 of the elongate device 44 through the lumen 18 of the device 10 by directing the distal end 26 of the elongate body 20 into the lumen 18 from the proximal end portion 14 of the device 10 (when the device 10 is not already attached onto the elongate body 20), out of the distal end portion 16 of the device 10, and further extend the elongate body 20 into the channel 30 of the scope 28. The user may continue to extend the elongate body 20 of the elongate device 44 along the channel 30 of the scope 28 while holding the device 10 in the first position 22. When the distal end 26 of the elongate body 20 reaches the desired location, the user may move the device 10 proximally with respect to the elongate body 20 such that the device 10 may disengage from the channel 30 of the scope 28 and may be coupled with respect to the elongate body 20. In some embodiments, the device 10 may be moved to the second position 24 and coupled to the component 38 (e.g., strain relief) attached to the proximal portion 36 of the elongate body 20 or coupled to the handle 48. Then the user may manipulate the elongate device 44 to extract the object without the distraction caused by a dangling device 10 that is freely movable along the elongate body 20 of the elongate device 44. After this use, the user may decouple the device 10 from the elongate body 20 such that the device may again be used for insertion of the elongate body 20 into a channel of a scope. The user may also remove (e.g., by peeling off) the device 10 from the elongate body 20 as needed or desired.

In certain aspects, the present disclosure also provides for a method of operating device components without reference to treatment of a human body, including method of guiding an elongate body into a channel of a scope using a device, comprising: placing the device in a first position with a distal end portion of the device inserted into a proximal end of a scope and into a portion of a channel of the scope; extending an elongate body through a lumen of the device by directing a distal end of the elongate body into the lumen from a proximal end portion of the device, out of the distal end portion of the device, and further extending the elongate body into the channel of the scope; holding the device in the first position as the elongate body continues to extend along the channel; moving the device with respect to the elongate body to a second position when the distal end of the elongate body reaches a desired location inside the channel; and coupling the device with respect to the elongate body.

The method may further include the device being slidably attached to the elongate body. In certain embodiments of the method, the lumen at the proximal end portion of the device has a first inner diameter and the lumen at the distal end portion of the device has a second inner diameter smaller than the first inner diameter. The method may further include coupling the device to a component attached to a proximal portion of the elongate body. In certain embodiments of the method, the device has a length such that when the device is coupled to the component attached to the elongate body in the second position, the device can provide support to the proximal portion of the elongate body. In certain embodiments of the method, the component attached to the proximal portion of the elongate body is a strain relief. The method may include that, when the device is in the first position, a first portion of the device is disposed inside the channel and a second portion of the device is disposed outside the channel.

In certain embodiments of the method, the device is configured to be peeled away from the elongate body. In certain embodiments of the method, the device is collapsible such that when the device is in the second position, the device can collapse to build column strength along the proximal portion of the elongate body. In certain embodiments of the method, the device has a length that is at least 5 cm. In certain embodiments of the method, the device comprises a slit or spiral cut. In certain embodiments of the method, the first portion of the device has a first length such that the elongate body can be guided, inside the lumen of the device, through at least one angled portion of the channel. In certain embodiments of the method, the proximal end portion of the device is a geometric cone. In certain embodiments of the method, the proximal end portion of the device is configured such that an outer diameter of the proximal end portion decreases in a distal direction at a non-linear rate.

While various embodiments of the present disclosure have been described, the present disclosure is not to be restricted except in light of the attached claims and their equivalents. One skilled in the relevant art will recognize that numerous variations and modifications may be made to the embodiments described above without departing from the scope of the present invention, as defined by the appended claims. Moreover, the advantages described herein are not necessarily the only advantages of the present disclosure and it is not necessarily expected that every embodiment of the present disclosure will achieve all of the advantages described.

## Claims

1. A device for guiding an elongate body into a channel of a scope, comprising:
a main body extending between a proximal end portion and a distal end portion; and
a lumen extending through the main body between the proximal end portion and the distal end portion,
wherein the device is configured to be slidably attached onto an elongate body such that the elongate body extends through the lumen and the device is movable between a first position and a second position with respect to the elongate body,
wherein when the device is disposed in the first position, a distal end of the elongate body is configured to be capable of being threaded into the lumen, out of the distal end portion of the main body, and into a channel of a scope, so that the distal end of the elongate body reaches a desired location inside the channel, and
wherein when the distal end of the elongate body reaches the desired location inside the channel, the device can be moved along the elongate body to the second position, wherein the device is releasably coupled with respect to the elongate body.

2. The device of claim 1, wherein the lumen at the proximal end portion of the main body has a first inner diameter and the lumen at the distal end portion of the main body has a second inner diameter smaller than the first inner diameter.

3. The device of claim 1, wherein the device has a funnel-shaped configuration at the proximal end portion.

4. The device of claim 2 or 3, wherein when the device is in the second position, the device is releasably coupled to a component attached to a proximal portion of the elongate body.

5. The device of claim 4, wherein the device has a length such that when the device is coupled to the component attached to the elongate body in the second position, the device can provide support to the proximal portion of the elongate body.

6. The device of claim 4 or 5, wherein the component attached to the proximal portion of the elongate body is a strain relief.

7. The device of any of claims 1 through 6, wherein when the device is in the first position, a first portion of the device is disposed inside the channel and a second portion of the device is disposed outside the channel.

8. The device of any of claims 1 through 7, wherein the device has a length that is at least 5 cm.

9. The device of any of claims 1 through 8, further comprising a slit or spiral cut on the main body.

10. The device of any of claims 1 through 8, wherein the device is configured to be peeled away from the elongate body.

11. The device of any of claims 4 through 6, wherein the device is collapsible such that when the device is in the second position, the device can collapse to build column strength along the proximal portion of the elongate body.

12. The device of claim 7, wherein the first portion of the device has a first length such that the elongate body can be guided, inside the lumen of the device, through at least one angled portion of the channel.

13. The device of any of claims 1 through 12, wherein the proximal end portion of the main body is a geometric cone.

14. The device of any of claims 1 through 12, wherein the proximal end portion of the main body is configured such that an outer diameter of the proximal end portion decreases in a distal direction at a non-linear rate.
